# EUROPEAN PATENT APPLICATION

(11) **EP 3 572 082 A1**
(43) Date of publication of application: **27.11.2019**
(21) Application number: 18742289.4
(22) Date of filing: 19.01.2018
(51) Int. Cl.: A61K 31/4709, A61P 35/00

(54) **USES OF EGFR/HER2 INHIBITOR COMBINED WITH PYRIMIDINE-TYPE ANTI-METABOLIC DRUG**

(30) Priority: 22.01.2017 CN 201710052960
(71) Applicant: Jiangsu Hengrui Medicine Co. Ltd., Jiangsu 222047 (CN)
(72) Inventor: ZOU, Jianjun, Lianyungang Jiangsu 222047 (CN); CAO, Guoqing, Lianyungang Jiangsu 222047 (CN); ZHU, Xiaoyu, Lianyungang Jiangsu 222047 (CN)
(74) Representative: Potter Clarkson
(86) International application number: PCT/CN2018/073386
(87) International publication number: WO 2018/133838

(57) **Abstract**

The present invention relates to uses of an EGFR/HER2 inhibitor combined with a pyrimidine-type anti-metabolic drug, and specifically, to uses of an EGFR/HER2 receptor tyrosine kinase inhibitor combined with pyrimidine-type anti-metabolic drug in the preparation of drugs for treating cancers.

## Description

### FIELD OF THE INVENTION

The present invention relates to a use of an EGFR/HER2 receptor tyrosine kinase inhibitor in combination with a pyrimidine antimetabolite in the preparation of a medicament for treating cancer.

### BACKGROUND OF THE INVENTION

Receptor tyrosine kinases (RTKs) are a class of transmembrane proteins involved in the signal transduction of growth factors, and comprise an extracellular domain containing a ligand binding site, a single-transmembrane hydrophobic alpha helix region and an intracellular domain having tyrosine protein kinase (RTK) activity. The occurrence and development of many tumors are closely related to the abnormal expression of tyrosine kinase. The existing data indicate that more than 50% of products of proto-oncogene and oncogene have tyrosine kinase activity, the abnormal expression of which will lead to disorder of cell proliferation regulation, thereby causing tumorigenesis, and is closely related to tumor invasion, metastasis and chemotherapy resistance. Receptor tyrosine kinase includes epidermal growth factor (EGF) receptor, fibroblast growth factor (FGF) receptor, vascular endothelial growth factor (VEGF) receptor and the like.

The EGFR family includes four members, i.e., EGFR/HER1/erbB-1, HER2/erbB-2, HER3/erbB-3, and HER4/erbB-4. In the 1980s, scientists discovered that the amplification and overexpression of HER2 is related to a more aggressive tumor phenotype. So far, no ligand has been found to bind to the HER2 receptor. HER2 molecule promotes the occurrence and progression of tumor diseases mainly by forming heterodimers with other receptors of the EGFR family to further activate MAPK, JAK, PI3K, STAT3 pathways and the like. HER2 gene is less expressed in normal epithelial cells, and is amplified/overexpressed in more than 30% of human tumors including breast cancer, gastric cancer, lung cancer and the like. HER2 has been widely concerned as a drug target. With the standardization of HER2 amplification detection, targeted therapy of HER2-positive patients has become a hot topic of basic and clinical research (Freudenberg JA, Wang Q, Katsumata M, et al. The role of HER2 in early breast cancer metastasis and the origins of resistance to HER2-targeted therapies. Exp Mol Pathol. 2009 Aug; 87(1):1-11). A number of HER2-targeted drugs, such as trastuzumab, pertuzumab, T-DM1 and lapatinib, are currently commercially available worldwide. Lapatinib in combination with capecitabine is approved for the treatment of patients with HER2+ metastatic breast cancer who previously received trastuzumab and a taxane, separately or in combination (Lapatinib. Drugs@FDA, 2014).

Trastuzumab in combination with chemotherapy is currently the first-line standard regimen for recurrence/metastasis treatment in breast cancer diagnosis and treatment in China. Lapatinib in combination with capecitabine is one of the second-line standard regimens (Capecitabine, Xeloda®, Aibin®). For patients who can use the above standard regimens, recent disease control and long-term survival benefits are guaranteed, but drug withdrawal and drug changing are still inevitably required due to disease progression, drug resistance and adverse reactions (Fan L, Strasser-Weippl K, Li JJ, et al. Breast cancer in China. Lancet Oncol. 2014 Jun; 15(7):e279-89).

CN102471312A discloses a compound of following formula A (chemical name: (*E*)-N-[4-[[3-chloro-4-(2-pyridylmethoxy)phenyl]amino]-3-cyano-7-ethoxy-6-quinolyl] -3-[(2*R*)-1-methylpyrrolidin-2-yl]prop-2-enamide), and discloses that it has a potent inhibition effect on EGFR and HER2, and is expected to be useful in the treatment of EGFR and HER2-overexpressing cancers.

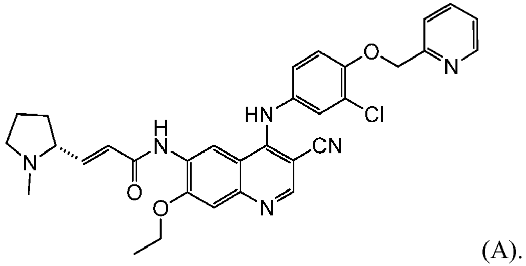

CN102933574A discloses a series of pharmaceutically acceptable salts of compound A. CN103974949A discloses a crystal form of the dimaleate salt of compound A.

The phase I clinical study in China shows that compound A has a certain effect on patients with HER2+ metastatic breast cancer (A phase I study for tolerability, safety, and pharmacokinetics of pyrotinib, a novel irreversible HER2 and EGFR inhibitor, in Chinese patients with HER2+ metastatic breast cancer. J Clin Oncol 33, 2015 (suppl; abstr e11596), NCT01937689).

However, the above documents do not disclose the use of compound A in combination with capecitabine for treating breast cancer, let alone what effect the combination can achieve.

### SUMMARY OF THE INVENTION

The present inventors surprisingly find that compound A or a pharmaceutically acceptable salt thereof in combination with a pyrimidine antimetabolite has a surprising efficacy in treating HER2+ metastatic breast cancer, thereby completing the present invention.

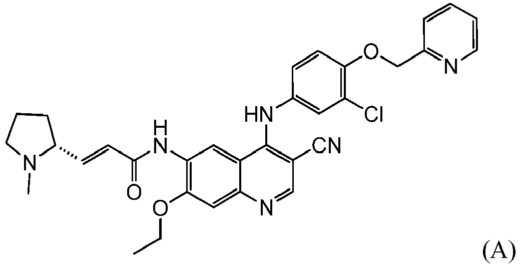

In one aspect, the present invention relates to a use of the compound of above formula A or a pharmaceutically acceptable salt thereof in combination with a pyrimidine antimetabolite in the preparation of a medicament for treating cancer. In a preferred embodiment of the present invention, the cancer is a HER2-overexpressing cancer, in particular a HER2-overexpressing breast cancer, such as a HER2-overexpressing recurrent/metastatic breast cancer. In the present invention, "HER2-overexpressing" has the same meaning as "HER2+", "HER2 positive" and "HER2-expression positive".

In the present invention, the pyrimidine antimetabolite can be selected from the group consisting of azacitidine, decitabine, fluorouridine, 5-fluorouracil, cytarabine, gemcitabine, tegafur and capecitabine, and preferably capecitabine.

The compound A can be used in the form of a pharmaceutically acceptable salt thereof, such as maleate salt, and preferably dimaleate salt.

In the actual administration, the daily administration dose of compound A or a pharmaceutically acceptable salt thereof, based on compound A, can be 100 mg to 1000 mg, and can be 100mg, 105mg, 110mg, 115mg, 120mg, 125mg, 130mg, 135mg, 140mg, 145mg, 150mg, 155mg, 160mg, 165mg, 170mg, 175mg, 180mg, 185mg, 190mg, 195mg, 200mg, 205mg, 210mg, 215mg, 220mg, 225mg, 230mg, 235mg, 240mg, 245mg, 250mg, 255mg, 260mg, 265mg, 270mg, 275mg, 280mg, 285mg, 290mg, 295mg, 300mg, 305mg, 310mg, 315mg, 320mg, 325mg, 330mg, 335mg, 340mg, 345mg, 350mg, 355mg, 360mg, 365mg, 370mg, 375mg, 380mg, 385mg, 390mg, 395mg, 400mg, 405mg, 410mg, 415mg, 420mg, 425mg, 430mg, 435mg, 440mg, 445mg, 450mg, 455mg, 460mg, 465mg, 470mg, 475mg, 480mg, 485mg, 490mg, 495mg, 500mg, 505mg, 510mg, 515mg, 520mg, 525mg, 530mg, 535mg, 540mg, 545mg, 550mg, 555mg, 560mg, 565mg, 570mg, 575mg, 580mg, 585mg, 590mg, 595mg, 600mg, 605mg, 610mg, 615mg, 620mg, 625mg, 630mg, 635mg, 640mg, 645mg, 650mg, 655mg, 660mg, 665mg, 670mg, 675mg, 680mg, 685mg, 690mg, 695mg, 700mg, 705mg, 710mg, 715mg, 720mg, 725mg, 730mg, 735mg, 740mg, 745mg, 750mg, 755mg, 760mg, 765mg, 770mg, 775mg, 780mg, 785mg, 790mg, 795mg, 800mg, 805mg, 810mg, 815mg, 820mg, 825mg, 830mg, 835mg, 840mg, 845mg, 850mg, 855mg, 860mg, 865mg, 870mg, 875mg, 880mg, 885mg, 890mg, 895mg, 900mg, 905mg, 910mg, 915mg, 920mg, 925mg, 930mg, 935mg, 940mg, 945mg, 950mg, 955mg, 960mg, 965mg, 970mg, 975mg, 980mg, 985mg, 990mg, 995mg, 1000mg, preferably 240 to 560 mg, and more preferably 320 to 480 mg. For Asians, the daily administration dose can also be in the range from 240 to 400 mg, and particularly preferably 400 mg. The compound A or a pharmaceutically acceptable salt thereof can be administrated once a day. Preferably, compound A or a pharmaceutically acceptable salt thereof of the present invention is administrated after a meal, i.e., administrated within 30 minutes after a meal, and more preferably administrated within 30 minutes after breakfast. In a most preferred embodiment, a pharmaceutical composition of 400 mg of compound A or a pharmaceutically acceptable salt thereof is orally administrated daily after breakfast.

With respect to the pyrimidine antimetabolite, the conventional administration dose can be used, or the administration dose can be appropriately reduced relative to the conventional administration dose. For example, the administration dose of capecitabine can be 1000 mg/m², bid.

In particular, the cancer patient can be a patient who has failed treatment, such as a patient who has failed treatment with a taxane drug and/or an anthracycline drug. The anthracycline drug is selected from the group consisting of doxorubicin, epirubicin and the like; and the taxane drug is selected from the group consisting of paclitaxel, docetaxel and the like.

Of course, the cancer patient can also be subjected to HER2 targeted treatment, wherein the drug used in the treatment is selected from the group consisting of trastuzumab, pertuzumab and T-DM1.

Further, in an optional embodiment, the cancer patient has a relative increase in target lesion diameter of at least 20%, or has the appearance of one or more new lesions.

In an optional embodiment, the cancer patient has a relative reduction in target lesion diameter of at least 30%.

In an optional embodiment, the tumor patient has a relative increase in target lesion diameter of up to 20%, or has a relative reduction in target lesion diameter of up to 30%.

Preferably, the cancer patient is a patient who has failed treatment.

The present invention also provides a pharmaceutical composition comprising the compound of formula A or a pharmaceutically acceptable salt thereof and a pyrimidine antimetabolite, wherein preferably the pyrimidine antimetabolite is selected from the group consisting of azacitidine, decitabine, fluorouridine, 5-fluorouracil, cytarabine, gemcitabine, tegafur and capecitabine, and more preferably the pyrimidine antimetabolite is capecitabine.

In another aspect, the present invention provides a method for treating cancer, comprising administrating to a patient in need thereof the compound of formula A and a pyrimidine antimetabolite. In a preferred embodiment of the present invention, the cancer is a HER2-overexpressing cancer, in particular a HER2-overexpressing breast cancer, such as a HER2-overexpressing metastatic breast cancer. In the present invention, "HER2-overexpressing" has the same meaning as "HER2+" and "HER2-expression positive". The pyrimidine antimetabolite is selected from the group consisting of azacitidine, decitabine, fluorouridine, 5-fluorouracil, cytarabine, gemcitabine, tegafur and capecitabine, and referably capecitabine. Preferably, "HER2-expression positive" means that no less than 10% of tumor cells have an immunohistochemical staining intensity of 2+ and are positive deteremined by fluorescence in situ hybridization [FISH], or have an immunohistochemical staining intensity of 3+ [staining intensity range of 0 to 3].

The median PFS of the patient population treated with compound A in combination with a pyrimidine antimetabolite according to the present invention is more than 2 times, even more than 2.5 times the median PFS of the patient population treated with lapatinib in combination with capecitabine. The ORR of the former can be increased by more than 10% or even more than 20% compared with the ORR of the latter. Likewise, the median TTP of the former can be more than 2 times or even more than 2.5 times the median TTP of the latter. The median DoR of the former can be more than 1.5 times or even close to 2 times the median DoR of the latter.

The median PFS of the patient population treated with compound A in combination with a pyrimidine antimetabolite according to the present invention can be more than 3 months, and preferably more than 9, 10, 11, 12, 13, 14, 15, 16, 17 or 18 months. The median TTP of the patient population receiving the treatment can be more than 8 months, and preferably more than 9, 10, 11, 12, 13, 14, 15, 16, 17, 18 or 19 months. The median DoR of the patient population receiving the treatment can be more than 9 months, and preferably more than 11, 12, 13, 14, 15 or 16 months. The present invention also relates to the compound of formula A or a pharmaceutically acceptable salt thereof in combination with a pyrimidine antimetabolite for use as a medicament for treating cancer.

In the actual administration, compound A or a pharmaceutically acceptable salt thereof can be formulated together with a pharmaceutically acceptable carrier into a composition form well known in the art, such as a tablet, capsule, granule, injection and the like. The present invention also relates to a use of a pharmaceutical composition comprising compound A for the use or method of treatment as described above.

In the present invention, the compound of formula A or a salt thereof is administrated in combination with a pyrimidine antimetabolite. The term "combination" includes various situations in which the two drugs are administrated sequentially or simultaneously. The term "simultaneously" herein refers to the administration of the compound of formula A or a salt thereof in combination with the pyrimidine antimetabolite during the same administration cycle, for example, the two drugs are administrated within two days, or within one day. The term "sequential" administration includes situations in which the compound of formula A or a salt thereof in combination with the pyrimidine antimetabolite are administered respectively, in different administration cycles. These administration modes all belong to the combination of the present invention. Preferably, the combination of the present invention means that the two drugs are administrated simultaneously, i.e., both the compound of formula A or a salt thereof and the pyrimidine antimetabolite are administrated during one administration cycle. It is not excluded that the pyrimidine antimetabolite can be administrated for a period of time, and then discontinued for a period of time during one administration cycle, for example administrated for two weeks, and then discontinued for one week.

The term "failed treatment" as described in the present invention means that the subject has measurable cancer lesions at baseline, and is classified into progressive disease (PD) or intolerance according to the RECIST 1.1 efficacy assessment criteria.

The term "intolerance" as described in the present invention means that the treatment cannot continue due to the drug-induced adverse reactions.

### DESCRIPTION OF THE DRAWING

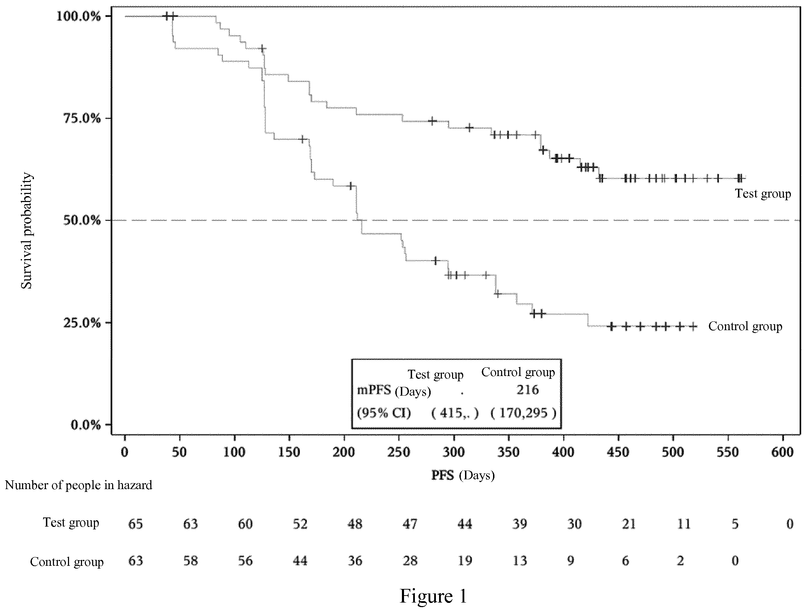

- Figure 1: shows the time-to-event PFS summary as of December 28, 2016.

### DETAILED DESCRIPTION OF THE INVENTION

**Example 1:** A randomized, open, parallel, multicenter, phase I/II clinical study of compound A in combination with capecitabine versus lapatinib in combination with capecitabine in treating HER2-expression positive metastatic breast cancer (NCT02422199)

### 1 Research objective and endpoints

### 1.1 Research objective

To compare the safety and efficacy of the regimen of compound A in combination with capecitabine versus the regimen of lapatinib in combination with capecitabine in treating HER2-expression positive metastatic breast cancer.

### 1.2 Endpoints

### 1. Main endpoints

Safety indicators: ECOG score, vital signs, physical examination, laboratory examination indicators (blood, urine and stool routine examination, blood biochemistry examination, pregnancy test and virological screening), ECG, echocardiography, adverse events (AE), according to NCI-CTC AE 4.0 criteria.

Effectiveness indicators: Objective response rate (ORR) within 12 cycles receiving the treatment regimen of the study, and the imaging evaluation are conducted by the research doctors at each center according to RECIST 1.1 criteria.

### 2. Secondary endpoints

Effectiveness indicators: Progression free survival (PFS), time to progression (TTP), and duration of response (DoR), according to RECIST 1.1 criteria.

### 2 Study design

### 2.1 General design

This study used a multi-center, randomized, open, dual-arm, positive drug parallel control design, and enrolled 128 breast cancer patients, who failed treatment with an anthracycline drug and a taxane drug and did not receive chemotherapy exceeding the second line after recurrence/metastasis. The patients were stratified according to whether they were previously treated with macromolecular antibodies targeting HER2, and then grouped randomly to the treatment group of compound A in combination with capecitabine (test group) or the treatment group of lapatinib in combination with capecitabine (control group) in a ratio of 1:1.

The subjects were administrated with the drugs continuously after enrollment, 1 cycle every 21 days, until progressive disease occured, the toxicity became intolerable, informed consent was withdrawn, or the investigator determined that the administration must be discontinued. During the study, all subjects were required to receive blood sampling for PK-PD study. For subjects providing tumor tissue sections, the wax blocks/slices (about 10 sheets) of the tumor tissue of the subjects could be collected once for molecular marker study. After completion of the treatment, the subjects would receive a safety follow-up until 28 days after the last administration, all adverse events restored to level I, or all adverse events were clinically stable, whichever comes later. The subjects exiting from the group due to Non-PD non-death causes would receive an efficacy follow-up until PD, starting to receive other anti-tumor drugs or death, whichever comes first. All subjects would receive a survival follow-up (OS data collection) until the subjects died, the subjects were lost to follow-up or the OS data collection was completed. This study used the EDC system to collect clinical data without interim analysis. Based on the FAS set, the previous safety and efficacy data were analyzed and summarized when the last subject completed the 12-cycle end efficacy evaluation or at least 96 subjects (75%) achieved PFS, whichever comes later. The survival follow-up (OS data collection) was completed when 106 subjects (83%) died or were lost to follow-up.

### 2.2 Random grouping and stratification factors of the study

Whether the patients were previously treated with macromolecular antibodies targeting HER2 was used as the stratification factor of the study. The subjects were grouped randomly to the group of compound A in combination with capecitabine (test group) or the group of lapatinib in combination with capecitabine (control group) in a ratio of 1:1.

Previously treated with macromolecular antibodies targeting HER2: If the patient has been treated with trastuzumab previously, the use in the neoadjuvant/adjuvant treatment phase should be ≥ months (in natural months), or the use after recurrence/metastasis phase should be ≥ cycles (for example: 21 days/cycle); if the patient has been treated simultaneously or sequentially with other macromolecular antibody drugs or antibody-conjugated drugs targeting HER2 such as pertuzumab, T-DM1 and the like, it does not not affect patient enrollment.

### 2.3 Administration regimen

Test group: 400 mg of compound A and 1000 mg/m² of capecitabine were orally administrated within 30 minutes after breakfast, and 1000 mg/m² of capecitabine was orally administrated 12 hours later.

Control group: 1250 mg of lapatinib was orally administrated before breakfast, 1000 mg/m² of capecitabine was orally administrated after breakfast, and 1000 mg/m² of capecitabine was orally administrated 12 hours later.

All drugs were orally administrated. Since eating has different effects on the above three drugs, it is necessary to pay special attention to the relationship between each administration for each drug and eating. The subjects were administrated with the drugs continuously after enrollment, until progressive disease occured, the toxicity became intolerable, informed consent was withdrawn, or the investigator determined that the administration must be discontinued.

### 2.4 Tumor imaging evaluation

The tumor imaging evaluation during administration was conducted by the research doctors at each center according to RECIST 1.1 criteria. The examination time point was determined from the beginning of the study treatment, regardless of the time of suspending administration due to toxicity during this period. The time window allowed for tumor imaging examination was ± days of treatment. The first evaluation was conducted on the 21^{st} day of the 2^{nd} cycle of administration, then one evaluation was conducted every two cycles until the 36^{th} cycle, and then one evaluation was conducted every four cycles after the 37^{th} cycle. The tumor imaging evaluation included neck, chest, and abdominal examinations. A brain examination was conducted during the baseline phase to exclude brain metastases. Pelvic examination, bone scan and the like can be added if necessary. The examination can be carried out by CT, spiral CT, PET-CT, MRI and the like. The imaging examination techniques used in the evaluations of the same patient at different time should be the same, and all imaging data should be stored. The subjects whose efficacy reached CR or PR for the first time would be reexamined and confirmed 4 weeks after the evaluation.

In the case of radiographically confirmed PD, the subject discontinued the trial and entered the follow-up phase. Other anti-tumor treatments cannot be carried out until PD was present.

With respect to the subjects exiting from the group due to Non-PD non-death causes, such as intolerance of toxicity, if the efficacy evaluation was not conducted within four weeks before exit, an efficacy evaluation should be conducted at the time of exit, and then the imaging examination was conducted in the same frequence until progressive disease (PD), receiving other anti-tumor treatments or death, whichever comes later.

All imaging examination data related to the efficacy evaluation would be recorded on a CD-ROM. The sponsor would decide whether to conduct a third-party evaluation or not based on the research situation.

### 3 Selection and exit of the subjects

This study planed to enroll 128 patients with HER2-expression positive (ICH and/or FISH) metastatic breast cancer (phase IV), who failed treatment with an anthracycline drug and a taxane drug treatment failure and did not receive chemotherapy exceeding the second line after recurrence/metastasis, and previously were treated or were not treated with macromolecular antibodies targeting HER2.

### 3.1 Enrollment criteria

The subjects must meet all of the following inclusion criteria to enroll in the trial:
1) Ages: 18 to 70 years old (including 18 and 70 years old);
2) ECOG physical status: level 0 to 1;
3) The expected survival period is not less than 12 weeks;
4) The patient has at least one measurable lesion (RECIST 1.1);
5) The patient has HER2-expression positive advanced breast cancer confirmed by histological or cytological examination ("HER2-expression positive") means that no less than 10% of tumor cells have an immunohistochemical staining intensity of 2+ and are positive deteremined by fluorescence in situ hybridization [FISH], or have an immunohistochemical staining intensity of 3+ [staining intensity range of 0 to 3]);
6) The patient has failed treatment with an anthracycline drug and a taxane drug and does not receive chemotherapy exceeding the second line after recurrence/metastasis, and previously were treated or were not treated with macromolecular antibodies targeting HER2;
7) The function of organs must meet the following requirements:
   - ANC function: 1.5×10⁹/L;
   - PLT function: 10×10⁹/L;
   - Hb ≥ 90 g/L;
   - TBIL ≤ 1.5LULN;
   - ALT and AST ≤ 1.5LULN; for patients with liver metastases, ALT and AST ≤ 5ST with liver;
   - BUN and Cr ≤ 1rN with liver and creatinine clearance ≥ 50 mL/min (Cockcroft-Gault formula);
   - LVEFtrof;
   - The QT interval corrected by the Fridericia method (QTcF) male < 450 ms, female < 470 ms.
8) The patients volunteers to participate in the study, sign informed consent, have good compliance and are willing to cooperate with follow-up.

"Previously failed treatment with an anthracycline drug" includes disease recurrence/progression after application in each treatment phase, such as neoadjuvant/adjuvant and post-recurrence/post-metastatic. If the application period of the anthracycline-containing regimen includes the neoadjuvant/adjuvant treatment phase, the cumulative dose of doxorubicin is at least equivalent to a total of 3 cycles of administration in 50 mg/m², but the cumulative dose should not exceed 400 mg/m². The cumulative dose of epirubicin is at least equivalent to a total of 3 cycles of administration in 90 mg/m², but the cumulative dose should not exceed 800 mg/m². If other anthracycline drugs are used, the dose will be converted based on this dose. If progressive disease occurs during the treatment with the anthracycline-containing regimen, it is not required that the previous dose must meet the above criteria.

"Previously failed treatment with a taxane drug" includes disease recurrence/progression after application in each treatment phase, such as neoadjuvant/adjuvant and post-recurrence/post-metastatic. The duration of treatment with the taxane drug should be ≥ cycles (for example: 21 days/cycle).

### 3.2 Exclusion criteria

The subjects cannot be included for any of the following conditions:
1) Presence of a third interstitial fluid (such as a large amount of pleural effusion and ascites) that cannot be controlled by drainage or other methods;
2) Presence of multiple factors affecting the administration and absorption of drugs, such as unable to swallow, chronic diarrhea and intestinal obstruction;
3) The subject has received radiotherapy, chemotherapy, hormone therapy, surgery or molecular targeted therapy within 4 weeks prior to enrollment; has received nitrosourea or mitomycin chemotherapy within 6 weeks prior to enrollment;
4) The subject has participated in other drug clinical trials within 4 weeks prior to enrollment;
5) The subject has treated with capecitabine within 6 months prior to enrollment; the subject has previously failed treatment with capecitabine (including progression in capecitabine therapy, or the duration of clinical efficacy after treatment < 3 months) or cannot tolerate capecitabine;
6) The patient has a brain tumor lesion diagnosed by brain CT or MRI; the patient has only bone or skin as the sole target lesion;
7) The subject was treated previously or is being treated with a tyrosine kinase inhibitor targeting HER2 (including lapatinib, natenatib and compound A, etc.);
8) The subject has previously suffered from other malignant tumors within 5 years, excluding cured cervical carcinoma in situ, cutaneous basal cell carcinoma or cutaneous squamous cell carcinoma;
9) The subject is receiving any other anti-tumor treatments at the same time;
10) The subject is allergic, or is known to have a history of allergies to the drug components of this regimen;
11) The patient is known to have dihydropyrimidine dehydrogenase (DPD) deficiency;
12) The subject has a history of immunodeficiency, including HIV positive, or has other acquired, congenital immunodeficiency disease, or has a history of organ transplantation;
13) The subject has suffered from any heart disease, including: (1) angina pectoris; (2) arrhythmia that needs drug treatment or clinically significant arrhythmia; (3) myocardial infarction; (4) heart failure; (5) any other heart diseases that are determined by the researcher to be unsuitable for participation in the trial and the like;
14) Female patients during pregnancy and lactation, female patients with fertility and positive baseline pregnancy test, or female patients of childbearing age who are unwilling to take effective contraceptive measures throughout the trial;
15) The patient has accompanying diseases that seriously endangers the safety of the patient or affects the completion of the study according to the investigator's judgment (including but not limited to high blood pressure, severe diabetes, active infections, thyroid diseases and the like);
16) The subject has bad habits such as alcoholism, smoking and the like (according to the investigator's judgment);
17) The subject has a clear history of neurological or psychiatric disorder, including epilepsy or dementia;
18) The investigator determines the patient who does not develop progressive disease after or during the treatment of the last anti-tumor regimen before enrollment according to imagining (RECIST1.1 criteria).

Intolerance is defined as the presence of hematologic toxicity that ≥ grade IV, or non-hematologic toxicity that ≥ grade III, or damage to major organs such as heart, liver and kidney that ≥ grade II during the treatment.

### 3.3 Exit criteria

The subject can withdraw informed consent and exit from the trial at any time.

The investigator can determine the subject's exit from the study in the following conditions:
1) Presence of any clinical adverse event, laboratory examination abnormality or other medical condition which causes the subject no longer benefits from continuous administration;
2) The subject meets any of the exclusion criteria and is unable to participate in the trial (including newly developed clinical indications during the trial or old problems that have not been discovered in time);
3) It is necessary to stop the trial considering from the perspective of medical ethics;
4) The subject has poor compliance, no longer receives administration or examination before the completion of all trials, or receives other anti-tumor treatments at the same time before the completion of the trial, or cannot complete the trial as planned.

### 3.4 Rejection criteria

1) The subject fails to complete at least 1 cycle of clinical trial according to the regimen, and the effectiveness evaluation cannot be conducted;
2) The subject severely violates the study regimen, and is not administered according to prescribed dose, method, and course of treatment.

### 3.5 Discontinuation criteria

This study can be discontinued or suspended early if there are adequate reasons. A written notice stating the reason for early discontinuation or suspension will be provided by the decision maker, and submitted to the investigator, sponsor, State Food and Drug Administration and relevant authorities. If the study is discontinued or suspended early, the principal investigator must immediately report the ethics committee and provide corresponding reason.

Reasons for discontinuation of this study include but are not limited to the following:
1) It is found in the trial that the clinical trial regimen has major errors, and it is difficult to evaluate the drug;
2) The sponsor requests discontinuation (due to such as funding reasons, management reasons and the like);
3) The State Food and Drug Administration or the ethics committee orders the discontinuation of the trial for certain reason.

### 4 Drugs for study

### 4.1 Summary of the drugs for study

### 4.1.1 Name and source

The dimaleate salt of compound A, formulated into tablets, produced and provided by Jiangsu Hengrui Medicine Co., Ltd.

Capecitabine tablets, being the commercially available generic drug Aibin® produced by Jiangsu Hengrui Medicine Co., Ltd.

Lapatinib mesylate tablets, being the commercially available drug Tykerb® produced by GlaxoSmithKline.

### 4.1.2 Dosage form and specification of the drugs

1) Dosage form: dimaleate salt of compound A, tablets, the dose is based on compound A.
   Specification: 40 mg, 60 mg, 200 mg.
   Packing: 400 mg dose group: two 200 mg tablets per bag;
   320 mg dose group: one 200 mg tablet and two 60 mg tablets per bag;
   240 mg dose group: one 200 mg tablet and one 40 mg tablet per bag;
2) Dosage form: Lapatinib tablets
   Specification: 250 mg/tablet
   Packing: 70 tablets/box
3) Dosage form: Capecitabine tablets
   Specification: 0.15 g/tablet, 0.5 g/tablet.
   Packing: 12 tablets/box.

### 4.1.3 Storage condition

Storage condition: Seal and store in a dry place below 25°C.

Validity: 24 months.

### 4.2 Administration method

### Test group:

Dimaleate salt of compound A: 400 mg once a day, orally administrated within 30 minutes after breakfast, and administrated continuously for 21 days as one cycle.

Capecitabine: 1000 mg/m², twice a day, orally administrated within 30 minutes after a meal (once in the morning and once in the evening with 12 hours interval, equal to a daily dose of 2000 mg/m², wherein the one in the morning is administrated in combination with compound A), administrated continuously on days 1-14, and 21 days as one cycle.

### Control group:

Lapatinib: 1250 mg once a day, orally administrated 1 hour before breakfast or 2 hours after a meal, and administrated continuously for 21 days as one cycle.

Capecitabine: 1000 mg/m², twice a day, orally administrated within 30 minutes after a meal (once in the morning and once in the evening with 12 hours interval, equal to a daily dose of 2000 mg/m²), administrated continuously on days 1-14, and 21 days as one cycle.

The above administration dose can be adjusted according to the adverse reaction of the subject according to the regimen. The subjects were administrated with the drugs continuously, until progressive disease occured, the toxicity became intolerable, informed consent was withdrawn, or the investigator determined that the administration must be discontinued. In case of drug missing, it is necessary to record in detail the time when the missed drug should be administered and the reason for drug missing, and then continue to administer the drug according to the regimen cycle, without supplement or periodic adjustment.

### 5 Study procedure

The study was divided into three parts: screening phase, trial phase and follow-up phase. The subjects entered the screening phase after signing the informed consent form. Baseline data was collected, including demographic characteristics, past medical history, combined administration, vital signs, physical examination, electrocardiogram, laboratory examination such as blood and urine routine examination and the like. After verifying compliance with the enrollment criteria, the random drug numbers were issued. The subjects were enrolled and entered the trial phase. The test group was treated with compound A in combination with capecitabine, and the control group was treated with lapatinib in combination with capecitabine. The drugs were administrated according to the administration mode prescribed by the regimen. Adverse events were closely observed and the efficacy was evaluated regularly during administration, until progressive disease occured, the toxicity became intolerable, informed consent was withdrawn, or the investigator determined that the administration must be discontinued. The subjects exited from the group. The subjects entered the follow-up phase after exit from the group. A safety follow-up was conducted until 28 days after the last administration, all adverse events restored to level I, or all adverse events were clinically stable, whichever comes later. The subjects exiting from the group due to Non-PD non-death causes would receive an efficacy follow-up. The tumor imaging evaluation was conducted according to the time points specified in the regimen beginning from the last tumor imaging evaluation during the study, until PD, starting to receive other anti-tumor drugs or death in subjects, whichever comes first. All subjects received a survival follow-up (OS data collection) until the subjects died, the subjects were lost to follow-up or the OS data collection was completed, whichever comes first.

### 6 Effectiveness evaluation

### 6.1 Main endpoint

Objective response rate (ORR) within the 12 cycles of the receiving regimen of the study: the proportion of patients whose tumor shrinks to a certain extent and remains for a certain period of time beginning from the subjects begin to receive the regimen of the study, until the day of efficacy evaluation at the end of the 12^{th} cycle or the day of the subjects exiting from the group due to progressive disease, whichever comes first, including cases of CR and PR. The response evaluation criteria in solid tumors (RECIST 1.1 criteria) are employed to assess tumor objective response. The subjects must have measurable tumor lesions at baseline. The efficacy assessment criteria are classified into complete response (CR), partial response (PR), stable disease (SD), and progressive disease (PD) according to the RECIST 1.1 criteria. The subjects who were first evaluated as CR and PR were confirmed after 4 weeks.

### 6.2 Secondary endpoints

Progression free survival (PFS): The time beginning from the random day until progressive disease (PD) confirmed by the first imaging assessment or death due to any cause. If the subject did not develop PD or death at the study cut-off date, or had received other anti-tumor treatments, the last efficacy evaluation result before the cut-off date or beginning date of other anti-tumor treatments (whichever comes first) was used as the truncation time.

Time to progression (TTP): The time beginning from the random day until progressive disease (PD) confirmed by the first imaging assessment. If the subject did not develop PD at the study cut-off date, or had received other anti-tumor treatments, the last efficacy evaluation result before the cut-off date or beginning date of other anti-tumor treatments (whichever comes first) was used as the truncation time. Duration of response (DoR): The overall duration of response refers to the time period from the first evaluation of CR/PR (whichever comes first) to recurrence or PD. SD duration refers to the time period from the time when the subject was enrolled in the study (random day) to PD.

The above indicators were evaluated according to the RECIST 1.1 criteria. The analysis of these indicators included the tumor evaluation results during the study treatment and follow-up phase. If the subject has several indicators that can be determined as PD, the one come first is used for TTP analysis. Recurrence, new lesions or death are considered to have reached the end of the study. Receiving other systemic anti-tumor treatments or anti-tumor treatments against the targeted lesion is also considered to be tumor progression.

### 7 Safety evaluation

Adverse events and serious adverse events were reported according to the study regimen. The safety of the compound was evaluated during the trial phase through adverse event recording, laboratory examinations, vital signs, physical examination, and electrocardiogram recording. During the trial, the conditions such as symptoms and signs of the subjects after administration should be closely observed. The occurred adverse events/responses should be addressed in a timely and effective manner to safeguard the safety and interests of the subjects. After the occurred adverse events/responses were addressed in a timely and effective manner, the type, symptoms, time of occurrence, degree (or grade), treatment method and outcomes thereof should be recorded. Analysis, evaluation, and statistics of the adverse events were then carried out as a basis for continued trials.

Each group of subjects was subjected to a comprehensive physical examination and laboratory examination during the trial, including measurement of body temperature, heart rate, respiratory rate, blood pressure, blood routine examination, urine routine examination, stool routine examination, blood biochemistry, 12-lead electrocardiogram, cardiac ultrasound and the like. The investigator should conduct a follow-up on the occurrence, clinical control and outcome of all adverse events until 28 days after the last administration, all adverse events restored to level I, or all adverse events were clinically stable, whichever comes later, and all adverse events were recorded in detail in the original medical record and CRF form.

### 8 Data analysis and statistical methods

The analysis population for this study includes the full analysis set (FAS), compliance set (PPS), safety set (SS), and PK set. The full analysis set is the main analysis set of the effectiveness analysis of this study.

In this study, unless otherwise stated, the data would be summarized using descriptive statistics in accordance with the following general principles.

The measurement data was summarized by mean, standard deviation, median, maximum and minimum.

The enumeration data was summarized by frequency and percentage. The survival rate was estimated from time-event data by Kaplan-Meier, and the survival curve was plotted. Blood drug concentration data was summarized by mean, standard deviation, coefficient of variation, median, maximum and minimum. The PK parameters were summarized by mean, standard deviation, coefficient of variation, geometric mean, geometric standard deviation, median, maximum and minimum.

The frequency and percentage were used to describe the ORR within 12 cycles receiving the treatment and to calculate the 95% confidence interval. The difference of objective response rate between the test group and the control group and the 95% confidence interval were calculated. A hypothesis test was conducted on the difference of ORR between the two groups using CMH statistic considering the central effect.

Analysis of progression-free survival (PFS): The median survival time was calculated using the Kaplan-Meier method, and the survival curves were plotted. Log-Rank test was used for comparison between groups. The Cox proportional hazard model was used to estimate the hazard ratio HR of the test group relative to the control group and its 95% confidence interval. Time to progression (TTP), duration of response (DoR), and analysis thereof were the same as PFS.

According to the current standards of Hengrui, the following safety analysis was carried out:
The incidence of adverse reactions was calculated by groups;
The frequency of adverse reactions was listed by systems, and the percentage was calculated;
A detailed list of cases of various adverse events;
A detailed list of cases of various adverse reactions;
The number of cases and the conversion rate of "normal converted to abnormal" or "increased abnormality" in laboratory indicators, electrocardiogram and physical examination after the trial;
Abnormal cases in laboratory indicators, electrocardiogram and physical examination, and a clinical explanation list.

Based on the above study regimen, 11 research centers nationwide completed the enrollment of a total of 128 subjects during the enrollment phase (June 8, 2015 to March 24, 2016). As of December 28, 2016, among 128 subjects, 76 (59%) subjects exited from the group for various reasons. The main reason for exit was progressive disease confirmed by imaging examination (compound A + capecitab 32.3% vs lapatinib + capecitabine 68.3%). The ORR of the group of compound A in combination with capecitabine was 79.7%, and the ORR of the group of lapatinib in combination with capecitabine was 56.5%. The median PFS of the control group was 216 days, and the PFS of the test group was still under follow-up. In the group of compound A in combination with capecitabine, the most common drug-related adverse events (>10%) include: diarrhea, hand-foot syndrome, emesis, nausea, loss of appetite and weakness; the most common laboratory-related adverse events include: decreased white blood cell count, decreased neutrophil count, increased ALT, increased total bilirubin and the like. The anti-tumor effect of compound A in combination with capecitabine on HER2+ advanced breast cancer is rapid, efficient, and sustainable, and the safety is tolerable. The specific data obtained are shown in Tables 1, 2 and 3 below:

**Table 1: Summary of the study results**

| | **Compound A + capecitabine (n=65)** | **Lapatinib + capecitabine (n=63)** |
|---|---|---|
| **Treatment duration (days)** | | |
| Median treatment time | 396 days | 214 days |
| Treatment time range (minimum - maximum) | 64 - 564 | 42 - 520 |

| **Exit/discontinuation of the trial n (%)** | | |
|---|---|---|
| Clinical progression | 1 (1.5%) | 3 (4.8%) |
| Imaging progression | 21 (32.3%) | 43 (68.3%) |
| Adverse events^{a} | 4 (6.2%) | 2 (3.2%) |
| Receiving other anti-tumor treatments | 0 | 0 |
| Withdrawal of informed consent | 0 | 1 (1.6%) |
| Violation of the trial regimen | 0 | 0 |
| Loss to follow-up | 0 | 0 |
| Death | 0 | 0 |
| Others ^{b} | 1 (1.5%) | 0 |

| | | |
|---|---|---|
| • EDC data totaled 76 cases of exit (27 vs 49) | | |

**Table 2: Adverse events related to the test drugs (positive, possible, undecidable) with an incidence of ≥**

| **Systematic organ classification Standard terms** | **Compound A + capecitabine (n=65)** | | **Lapatinib + capecitabine (n=63)** | |
|---|---|---|---|---|
| | **All grades** | **≥ grade III** | **All grades** | **≥ grade III** |
| **Gastrointestinal diseases** | | | | |
| Diarrhea | **60(92.3%)** | 8(12.3%) | **25(39.7%)** | 1(1.6%) |
| Emesis | **26(40.0%)** | 2(3.1%) | 12(19.0%) | 1(1.6%) |
| Nausea | **19(29.2%)** | 0 | **13(20.6%)** | 0 |
| Oral ulcer | 10(15.4%) | 0 | 8(12.7%) | 0 |
| Stomach ache | 8(12.3%) | 0 | 5(7.9%) | 0 |
| Oral mucositis | 5(7.7%) | 0 | 10(15.9%) | 1(1.6%) |
| Ventosity | 1(1.5%) | 0 | 5(7.9%) | 0 |

| **Skin and subcutaneous tissue diseases** | | | | |
|---|---|---|---|---|
| Palmar redness and pain syndrome | **42(64.6%)** | 10(15.4%) | **43(68.3%)** | 10(15.9%) |
| Pigment abnormality | 9(13.8%) | 0 | 3(4.8%) | 0 |
| Rash | 2(3.1%) | 0 | **13(20.6%)** | 1(1.6%) |

| **Metabolic and nutritional diseases** | | | | |
|---|---|---|---|---|
| Loss of appetite | **14(21.5%)** | 0 | 2(3.2%) | 0 |
| Hypocalcemia | 6(9.2%) | 0 | 1(1.6%) | 0 |
| Hypokalemia | 6(9.2%) | 0 | 1(1.6%) | 1(1.6%) |
| Hypertriglyceridemia | 6(9.2%) | 2(3.1%) | 3(4.8%) | 1(1.6%) |
| Hyperglycemia | 4(6.2%) | 0 | 1(1.6%) | 0 |

| **Systemic diseases and various reactions at the site of administration** | | | | |
|---|---|---|---|---|
| Weakness | 11(16.9%) | 1(1.5%) | 10(15.9%) | 0 |
| Fever | 4(6.2%) | 0 | 5(7.9%) | 1(1.6%) |

| **Infection and infective diseases** | | | | |
|---|---|---|---|---|
| Upper respiratory tract infection | 9(13.8%) | 1(1.5%) | 8(12.7%) | 0 |
| Nasopharyngitis | 5(7.7%) | 0 | 7(11.1%) | 0 |

| **Blood and lymphatic system diseases** | | | | |
|---|---|---|---|---|
| Anemia | 11(16.9%) | 1(1.5%) | 3(4.8%) | 0 |

| **Various neurological diseases** | | | | |
|---|---|---|---|---|
| Dizziness | 6(9.2%) | 0 | 6(9.5%) | 0 |

| **Respiratory system, chest and mediastinal diseases** | | | | |
|---|---|---|---|---|
| Cough | 4(6.2%) | 0 | 6(9.5%) | 0 |

| **Various examinations** | | | | |
|---|---|---|---|---|
| Decreased white blood cell count | **23(35.4%)** | 1(1.5%) | 1**4(22.2%)** | 2(3.2%) |
| Decreased neutrophil count | **21(32.3%)** | 2(3.1%) | **13(20.6%)** | 1(1.6%) |
| Increased alanine aminotransferase | **17(26.2%)** | 1(1.5%) | 12(19.0%) | 1(1.6%) |
| Increased blood bilirubin | 12(18.5%) | 0 | **22(34.9%)** | 3(4.8%) |
| Increased aspartate aminotransferase | 11(16.9%) | 2(3.1%) | 12(19.0%) | 1(1.6%) |
| Increased conjugated bilirubin | 7(10.8%) | 0 | **17(27.0%)** | 2(3.2%) |
| Increased blood triglyceride | 5(7.7%) | 0 | 6(9.5%) | 1(1.6%) |
| Increased blood unconjugated bilirubin | 5(7.7%) | 0 | **15(23.8%)** | 2(3.2%) |
| Decreased platelet count | 4(6.2%) | 1(1.5%) | 1(1.6%) | 0 |
| Decreased lymphocyte count | 4(6.2%) | 0 | 1(1.6%) | 0 |

**Table 3: Optimal efficacy evaluation**

| **Optimal efficacy evaluation** | **Compound A + capecitabine (n=64)** | **Lapatinib + capecitabine (n=62)** |
|---|---|---|
| Complete response (CR) | 3 (4.7%) | 1 (1.6%) |
| Partial response (PR) | 48 (75.0%) | 34 (54.8%) |
| Stable disease (SD) | 13 (20.3%) | 22 (35.5%) |
| Progressive disease (PD) | 0 | 5 (8.1%) |
| Objective response rate (ORR) | **51 (79.7%)** | **35 (56.5%)** |

The clinical trial report of the phase I/II clinical trial of this embodiment was completed in June 2017. A total of 128 subjects were randomly grouped in the study, wherein 65 subjects were grouped to the compound A group, and 63 subjects were grouped to the lapatinib group. As of March 15, 2017, 34 (52.3%) subjects in the compound A group and 55 (87.3%) subjects in the lapatinib group had discontinued the study, respectively.

### Effectiveness results:

According to the tumor evaluation data determined by the investigator (as of March 15, 2017, RECIST v1.1 criteria), ORR, PFS, TTP and DoR results within 12 cycles were obtained.

### ORR within 12 cycles (main endpoint):

The ORR of the compound A group was 78.5% (95%CI: 68.5%, 88.5%), and the ORR of the lapatinib group was 57.1% (95%CI: 44.9%, 69.4%). Compared with the lapatinib group, the ORR of the compound A group increased by 21.3% (95%CI: 4.0%, 38.7%), and the difference was statistically significant (P = 0.01), indicating that the compound A group was superior.

### PFS:

Compared with the lapatinib group, the median PFS of the compound A group was significantly prolonged (the median PFS of the compound A group was 18.1 months [95%CI: 13.88 months, not reached], the median PFS of the lapatinib group was 7.0 months [95%CI: 5.62 months, 9.75 months], P<0.0001). Similarly, relative to the lapatinib group, the hazard of progressive disease or death of the compound A group was reduced by 63.7% (HR=0.363, 95%CI: 0.228, 0.579).

### TTP:

Compared with the lapatinib group, the median TTP of the compound A group was significantly prolonged (the median TTP of the compound A group was 19.5 months [95%CI: 13.88 months, not reached], and the median TTP of the lapatinib group was 7.0 months [95%CI: 5.62 months, 9.75], P<0.0001).

### DoR:

Compared with the lapatinib group, the DoR of the compound A group was significantly prolonged. Median DoR: the median DoR of the compound A group was 16.7 months (95%CI: 12.50 months, not reached), and the median DoR of the lapatinib group was 8.4 months (95%CI: 4.73 months, 13.82 months).

### Safety results:

In general, the lapatinib group and the compound A group were well tolerated. According to the systemic organ classification, the most common adverse events in the two groups were gastrointestinal system diseases, various examinations, and skin and subcutaneous tissue diseases. No safety events other than the safety report of the previous clinical study were found in this study.
- Almost all patients in the two groups had at least one all-cause adverse event (100% in the compound A group and 98.4% in the lapatinib group), wherein the subjects with drug-related adverse events accounted for 100% and 95.2%, respectively.
- The most common drug-related adverse events in the compound A group were diarrhea (96.9% vs 44.4%), palmar redness and pain syndrome (PPE) (78.5% vs 82.5%), emesis (47.7% vs 20.6%), decreased white blood cell count (46.2% vs 34.9%), nausea (40.0% vs 23.8%), increased blood bilirubin (30.8% vs 49.2%), and the like.
- During the treatment, the incidence of ≥ grade III adverse event in the compound A group was higher than that in the lapatinib group (63% vs 48%). The number and proportion of of subjects with ≥ grade adverse events in the compound A group and the lapatinib group were arranged in the order from high to low as follows: diarrhea: 10 cases (15.4%) and 3 cases (4.8%), decreased neutrophil count: 6 cases (9.2%) and 2 cases (3.2%), decreased white blood cell: 5 cases (7.7%) and 1 case (1.6%), and emesis: 3 cases (4.6%) and 1 case (1.6%). A possible cause of this difference was that the drug exposure time of the compound A group was significantly longer than that of the lapatinib group (2 folds) (median or mean exposure time results). In addition, ≥ grade III increased blood bilirubin, increased conjugated bilirubin and increased blood triglycerides only occured in the lapatinib group.
- The incidence of serious adverse events was comparable between the two groups (5/65, 7.7% in the compound A group; 4/63, 6.3% in the lapatinib group).
- The proportion of deaths due to adverse events was comparable between the two groups, 2 cases (3.1%) and 1 case (1.6%), respectively.
- The proportion of exit from the trial early due to adverse events was comparable between the two groups, 4 cases (6.2%) and 3 cases (4.8%), respectively.
- The vast majority of adverse events focused specifically by the study was of grade 1/2, and was clinically controllable. Diarrhea was the most common adverse event, and the incidence of grade III diarrhea was 15.4% in the compound A group and 4.8% in the lapatinib group, respectively. There were no grade IV diarrhea in either group or no cases of exit from the trial or death due to diarrhea in either group. In the compound A group, 88% of the subjects were able to tolerate. In the lapatinib group, 41% of the subjects were able to tolerate. There was no need to suspend or decrease the dose of compound A.

### Conclusion:

The present study was a randomized, open, positive control, multicenter, phase II clinical study of compound A in combination with capecitabine versus lapatinib in combination with capecitabine in treating HER2-positive advanced breast cancer. The study results indicated that:
- Compared with lapatinib in combination with capecitabine, the patients who failed treatment with an anthracycline drug and a taxane drug and did not receive chemotherapy exceeding the second line after recurrence/metastasis can benefit significantly from the treatment of compound A in combination with capecitabine. The treatment of compound A in combination with capecitabine improved the patient's objective response rate (ORR), prolonged the patient's progression-free survival (PFS) and time to progression (TTP), and improved the duration of response (DoR).
- The efficacy was improved meanwhile the tolerance of the patients was good. Although the incidence of gastrointestinal tract, skin reaction, and hematologic toxicity is higher in the compound A group, it can be controlled by suspension of administration, dose decrease, and symptomatic treatment.

## Claims

1. Use of a compound of formula A or a pharmaceutically acceptable salt thereof in combination with a pyrimidine antimetabolite in the preparation of a medicament for treating cancer,

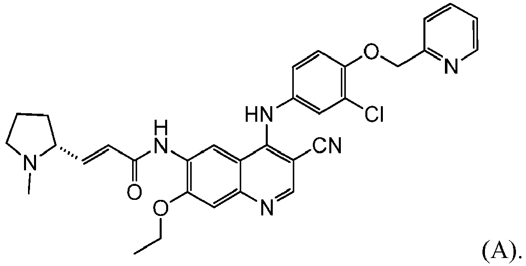

2. The use according to claim 1, wherein the cancer is a HER2-overexpressing cancer.

3. The use according to claim 2, wherein the cancer is a breast cancer.

4. The use according to claim 2, wherein the cancer is a recurrent/metastatic breast cancer.

5. The use according to claim 1, wherein the pyrimidine antimetabolite is selected from the group consisting of azacitidine, decitabine, fluorouridine, 5-fluorouracil, cytarabine, gemcitabine, tegafur and capecitabine, and preferably capecitabine.

6. The use according to claim 1, wherein the pharmaceutically acceptable salt of compound A is a maleate salt, and preferably a dimaleate salt.

7. The use according to claim 1, wherein the daily administration dose of compound A or a pharmaceutically acceptable salt thereof, based on compound A, is 100 mg to 1000 mg, preferably 240 to 560 mg, and more preferably 320 to 480 mg.

8. The use according to claim 1, wherein the cancer patient is a patient who has failed treatment with a taxane drug and/or an anthracycline drug.

9. The use according to claim 1, wherein the cancer patient is subjected to HER2 targeted treatment, wherein the drug used in the treatment is selected from the group consisting of trastuzumab, pertuzumab and T-DM1.

10. A pharmaceutical composition, comprising the compound of formula A or a pharmaceutically acceptable salt thereof and the pyrimidine antimetabolite according to claim 1, wherein preferably the pyrimidine antimetabolite is selected from the group consisting of azacitidine, decitabine, fluorouridine, 5-fluorouracil, cytarabine, gemcitabine, tegafur and capecitabine, and more referably the pyrimidine antimetabolite is capecitabine.

11. A method for treating cancer, comprising administrating to a patient in need thereof a compound of formula A and a pyrimidine antimetabolite,

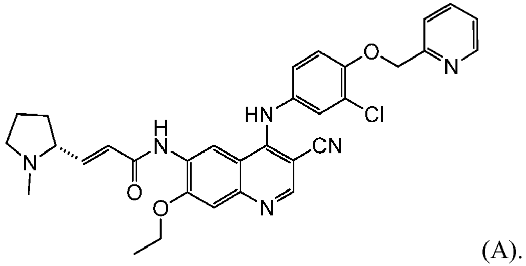

12. The method according to claim 11, wherein the cancer is a HER2-overexpressing cancer.

13. The method according to claim 12, wherein the cancer is a breast cancer.

14. The method according to claim 12, wherein the cancer is a recurrent/metastatic breast cancer.

15. The method according to claim 11, wherein the pyrimidine antimetabolite is selected from the group consisting of azacitidine, decitabine, fluorouridine, 5-fluorouracil, cytarabine, gemcitabine, tegafur and capecitabine, and preferably capecitabine.
